# EUROPEAN PATENT SPECIFICATION

(11) **EP 0 862 411 B1**
(45) Date of publication and mention of the grant of the patent: **09.10.2002**
(21) Application number: 96936979.2
(22) Date of filing: 25.10.1996
(51) Int. Cl.: A61K 7/48, A61K 7/025

(54) **METHODS FOR IMPROVING THE PERFORMANCE OF LONG-WEARING COSMETIC PRODUCTS**
VERFAHREN ZUR VERBESSERUNG DER LEISTUNG VON KOSMETISCHEN PRODUKTEN FÜR LANGEN GEBRAUCH
PROCEDES D'UTILISATION VISANT A AMELIORER LES PERFORMANCES DE PRODUITS COSMETIQUES A LONGUE TENUE

(30) Priority: 07.11.1995 US 6273 P; 13.12.1995 US 8552 P
(43) Date of publication of application: 09.09.1998
(73) Proprietor: THE PROCTER & GAMBLE COMPANY, Cincinnati, Ohio 45202 (US)
(72) Inventor: DRECHSLER, Lee, Ellen, Cincinnati, OH 45220 (US); RABE, Thomas, Elliot, Baltimore, MD 21229 (US); SMITH, Edward, Dewey, III, Mason, OH 45040 (US); DOHMAE, Terutomo, Yasu-gun, Shiga 520-23 (JP); HINES, Christina, Huston, TX 77054 (US)
(74) Representative: Wilding, Richard Alan
(86) International application number: US9617145
(87) International publication number: WO97017057

(56) References cited:
- EP-A- 0 519 727
- DE-A- 3 837 473
- DE-A- 4 025 040
- PATENT ABSTRACTS OF JAPAN vol. 13, no. 24 (C-561) [3372] , 19 January 1989 & JP 63 230618 A (KAO), 27 September 1988,

## Description

### TECHNICAL FIELD

The invention is for compositions and method for using said compositions to improve the performance of long-wearing cosmetic products. These compositions and methods for using such compositions enable the user to significantly enhance the attributes of long-wearing cosmetic products without compromising their primary advantages.

### BACKGROUND ART

Compositions used to enhance cosmetic products are known in the art. Such compositions include those that are applied over top compositions such as lipstick to provide attributes such as gloss, lubricity and transfer-resistance of the cosmetic product they are applied over. These enhancement products utilize a variety of polymeric fluids and film forming technologies. For example, acrylic film-formers that are incorporated in lipstick overcoat products such as CSI Incorporated's "Sealed with a Kiss" are delivered in a volatile vehicle, alcohol, which is spread over the lipstick surface.

Alternative topcoat products to those described above are disclosed in Japanese Patent Application Number HEI 5[1993]-221829, published August 31, 1993. Said overcoats are reputed to exhibit improved durability of makeup effect, suppression of color transfer, and improved applicability. Said topcoats comprise from 0.2 to 25% of silica powder and/or alumina powder and from 75% to 99.8% of a perfluoropolyether of general formula: 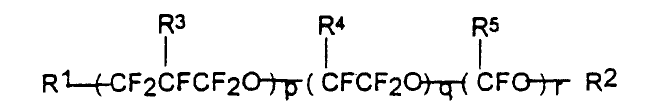 wherein R¹ though R⁵ are independent fluorine atoms, perfluoroalkyl groups, or oxyperfluoroalkyl groups; the value of p, q, and r is at least zero; wherein the perfluoropolyether molecular weight is from about 500 to about 10,000, wherein P, Q and R may be equal, but, not zero. The preferred perfluoropolyether disclosed therein is a commercially available product known as Fomblin HC-04, HC-25, and HC-R available from Montefluosu of Milano, Italy.
DE 3837473 discloses the use of from 3% to 60% by weight of sucrose fatty acid ester wherein the fatty acid moiety has from 2 to 6 carbon atoms to increase the adhesiveness of cosmetic compositions.
DE 4025040 discloses an oily composition for external application comprising (a) a dextrin fatty acid ester, (b) a glycerol fatty acid ester having a melting point of higher than 20°C, and (c) a cosmetically acceptable oil which is liquid at 20°C.
JP63230618 discloses a cosmetic composition comprising a mixture of a certain polyethylene wax and a low-viscosity fatty acid tri- or diglyceride.
EP-A-0519727 discloses a cosmetic oleogel suitable for topical application to the human body surface, which comprises (a) from 5 to 95% by weight of a solvent chosen from certain polyol fatty acid polyesters, (b) from 5 to 70% by weight of a cosmetically acceptable hydrophobic polymer thickener substantially free from cross linking.

While such compositions may provide certain advantages, it has been found that they often disrupt the primary advantages of the cosmetic products they are applied over. For example, cosmetic products compromise their gloss or feel attributes in order to improve the long wear properties provided by the composition that is applied over top the cosmetic product. Alternately, cosmetic products must sacrifice long wear properties in order to improve the gloss and or feel attributes provided by the such compositions.

### SUMMARY OF THE INVENTION

The present invention is for methods for using complementary compositions with cosmetic products having a solubility parameter less than or equal to 8.5 (calories /cm³) ^{1/2} in order to improve the overall performance associated with the cosmetic product. These complementary compositions comprise oils having a C log P value greater than or equal to 13.

Additionally, the present invention covers a method of improving transfer resistant, flexible film-forming cosmetic product wherein said method comprises the steps of:
a. applying a transfer resistant, flexible film-forming cosmetic product wherein said cosmetic product has a solubility parameter less than or equal to 8.5 (calories /cm³)^{1/2};
b. allowing said cosmetic product to dry; and
c. applying over said cosmetic product a complementary composition wherein said composition has a C log P value greater than or equal to 13.

### BACKGROUND OF THE INVENTION

It is best that in supplementing the benefits of a cosmetic product with a complementary composition, the primary benefits attributable to the cosmetic product should not be compromised. In context of film-forming cosmetic products, the complementary compositions should be sufficiently incompatible with the cosmetic film wherein it is not disrupted when the complementary composition is applied. By incompatible it is meant that the complementary composition comprises specific components that do not disrupt the film formed after application of the cosmetic product.

### a. Transfer-resistant, Flexible Film-forming Cosmetic Products

The complementary compositions of the present application may be used in conjunction with all types of cosmetic products wherein it is desirable to provide additional attributes. In the case of lip products, such attributes include gloss, shine and lubricity.

Lip cosmetic products are well known in the art and can encompass a number of different formulations in order to provide both cosmetic and skin care benefits to the skin. One benefit that has been most often sought by consumers, particularly in lip cosmetic product, is increased or "long" wear.

Long wearing cosmetic products are considered by some to be those that are resistant to blotting on to another object that comes in contact with the cosmetic product; for example, resistance to lip composition coming off onto table wear such as cups and napkins. However, other factors found to be critical in predicting long wear is the ability of the cosmetic product to be flexible and resistant to solvents such as food oils once applied to the skin. Such compositions comprise organosiloxane resins, fluid diorgansiloxane polymers, and a volatile carrier wherein the film formed upon application of the cosmetic is substantially transfer-resistant and flexible wherein the cosmetic product has surprisingly increased wear. Complementary compositions that are sufficiently incompatible with the cosmetic product are disclosed therein as useful with such cosmetic products. "Sufficiently incompatible" is disclosed as meaning that the application of the complementary composition does not disrupted the film formed by such cosmetic products.

The cosmetic products used in conjunction with the complementary composition of the present invention has a solubility parameters less than or equal to about 8.5 (calories /cm³) 1/2 on the Hildebrand scale. In general, the solubility parameter is a function of the cohesive energy of the materials or the cosmetic product comprising said materials. Cohesive energy is simply an attractive force that is dependent on the electro-negativities of the atoms making up a molecule and serves as the basis for properties such as viscosity, adhesion, miscibility and even the boiling point. Some materials, like water, have high cohesive energy; some, like oil, have low cohesive energy. Highly cohesive ingredients are "polar", while those less cohesive are oily or "non-polar". Hildebrand developed a method for deriving the solubility parameter from the boiling point, molecular weight and specific gravity of a material; J.H. Hildebrand, J.M. Prausnitz and R.L. Scott, Regular and Related Solutions, New York; Van Nostrand Reinholdt (1950). This Hildebrand solubility parameter is published for many cosmetic and pharmaceutical materials in the Cosmetic Bench Reference, Carol Stream IL, Allured Publishing (1992) and in A.F. Barton, Handbook of Solubility Parameters and Other Cohesion Parameters, 2nd ed., Boca Raton; CRC Press (1992).

### b. Complementary Compositions

The complementary composition of the present invention comprises oils and may take forms ranging from solid to liquids. Regardless of the form, the complementary compositions of the present invention the C log P value of the composition is equal to or greater than 13, preferably greater than or equal to 17, and most preferably greater than or equal to 20.

The C log P value of the composition determines whether the complementary composition is sufficiently incompatible with the cosmetic product in order to improve the cosmetic products performance. The value P is the octanol/water partitioning coefficient of the oils comprising the complementary composition. The octanol/water partitioning coefficient is the ratio between the compositions equilibrium concentration in octanol and in water. Since the values of the octanol/water partitioning coefficient are high, they are more conveniently given in the form of the logarithm to the base 10, or log P.

The log P values above are calculated using the "C log P" program available from Daylight CIS. This calculated logarithm of P is based on the fragment approach of Hansch and Leo (cf., A. Leo, in Comprehensive Medicinal Chemistry, Vol. 4, C. Hansch, P. G. Sammens, J. B. Taylor and C. A. Ransden, Eds., p. 295, Pergamon Press, 1990). The fragment approach is based on the chemical structure of each oil ingredient, and takes into account the numbers and types of atoms, the atom conductivity, and chemical bonding. The C log P values are the most reliable and widely used estimates for this physiochemical property.

The oil component of the complementary composition is the predominate ingredient of the composition. Typically the oil content of the complementary composition is at least about 55%, preferably about 65% and most preferably about 75% by weight of the composition. As such the aggregate C log P value for all the non-solid molecular entities comprising the complementary composition is about that of the oil alone. The complementary compositions of the present invention comprises oils selected from the group consisting of polyol fatty acid polyester, triglycerides, fluid synthetic polymers and mixtures thereof.

### Polyol Fatty Acid Polyesters

Polyol fatty acid polyesters are fatty acid polyesters derived from any aliphatic or aromatic polyol that has at least 4 free hydroxyl groups, of which at least 80% of these free hydroxy groups are then esterified with one or more fatty acids having from 8 to 22 carbon atoms.

The polyol from which the polyol fatty acid polyesters are derived are preferably chosen from sugar polyols that comprise mono-, di, and polysaccharides. Preferred examples of monosaccharide sugar polyols include:
Pentose sugar polyols such as D-ribose, D-arabinose, D-xylose, D-lyxose, D- ribulose and D-xylulose;
Hexose sugars polyols such as D-allose, D-altrose, D-glucose, D-mannose, D-gulose,
D-idose, D-galactose, D- talose, D-fructose, D-sorbose and D-tagatose;
Heptose sugar polyols such as D-mannoheptulose and D-sedoheptulose;

The polyol from which the polyol fatty acid polyesters are derived can also be chosen from disaccharides such as maltose, lactose, celloblose, sucrose, trehalose, gentioblose, meliblose and primeverose.

The polyol from which the polyol fatty acid polyesters are derived can also be chosen from tri-saccharides such as gentianose and raffinose.

The polyol from which the polyol fatty acid polyesters are derived can alternatively be chosen from sugar alcohols such as D-mannitol, D-sorbitol, D-ribitol, D-erithritol, D-lactitol and d-xylitol.

The polyol from which the polyol fatty acid polyesters are derived can also alternatively be chosen from sugars such as methyl glucoside and inositol. The preferred sugar polyol is sucrose. The sucrose polyol fatty acid esters or SPEs are disclosed in the priority document cited in the specification and are derived from sucrose and vegetable oil. This has been extensively disclosed in the patent literature in context of a non-digestible oils, including but not limited to U. S. Patents 3,600,186, issued August 17, 1971; 4,005,195, issued January 25, 1977; 4,005,196, issued January 25, 1977; all assigned to the Procter & Gamble Company.

The fatty acids that are employed to form the polyol fatty acid polyesters disclosed herein can be individual free fatty acids having from 8 to 24, preferably 16 to 22 carbon atoms in the fatty acid molecule. These fatty acids can be saturated or unsaturated, linear or branched chain fatty acids.

### Fats and Oils

Fats and oils useful in the present invention are triacylglycerides or triglycerides formed by an esterification reaction of fatty acids with glycerol. While the distinction between fats and oils is arbitrary, fats are typically considered solid or plastic at room temperature while oils are liquid under these same conditions. The fatty acids which are subsequently esterified to form triglyceride fats and oils are most usually derived form marine, animals and plant sources. For more information regarding triglyceride oils, their sources and processing, refer to Bailey, "Industrial Oil and Fats Products", Interscience Publications.

At least 90% of the ester substitution on the triglyceride backbone has carbon chain lengths of at least 12. The oils frequently are hydrogenated to some extent to deter rancidity. Such triglycerides include plant derived oils such as soy bean oil, castor bean oil, olive oil, sunflower oil, almond oil, peanut oil, canola oil, corn oil, other similarly related vegetable oils and mixtures thereof.

### Synthetic Polymer Oils

Synthetic polymer oils are useful in the present invention. Said synthetic polymer oils are liquid at room temperature and include glycerin/diethylene glycol/adipate crosspolymers, available as Lexorez 100 from Inolex Chemical Company.

### Optional Ingredients

There are a great number of other ingredients approved for use in the cosmetic art that may be used in compositions of the present invention. Such ingredients are those approved for use in cosmetics and can be found listed in reference books such as the CTFA Cosmetic Ingredient Handbook, Second Edition, The Cosmetic, Toiletries, and Fragrance Association, Inc. 1988, 1992. Said materials may be used provided their inclusion does not significantly disrupt the film formed once the cosmetic product has been applied to the skin. Said ingredients include waxes, fragrances, flavor oils, skin care ingredients such as sunscreen, emulsifiers and the like. Hypoallergenic compositions can be made into the present invention where said compositions do not contain fragrances, flavor oils, lanolin, sunscreens, particularly PABA, or other sensitizers and irritants. The additional ingredients that are added should not lower the C Log P values for the composition as a whole to less than 13. The following is a discussion of a non-exclusive group of such additional ingredients:

While the complementary composition is typically transparent, colorants including pigments and particulates such talc and mica may be used to add desirable effects to the cosmetic product. Colorants suitable for use herein are all inorganic and organic colors/pigments suitable for use in lip composition compositions. These include are usually aluminum, barium or calcium salts or lakes. Lakes are either a pigment that is extended or reduced with a solid diluent or an organic pigment that is prepared by the precipitation of a water-soluble dye on an adsorptive surface, which usually is aluminum hydrate. A lake also forms from precipitation of an insoluble salt from an acid or basic dye. Calcium and barium lakes are also used herein.

Preferred lakes of the present invention are Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake and Blue 1 Aluminum Lake, Red 6 Barium Lake, Red 7 Calcium Lake.

Other colors and pigments can also be included in the lip compositions, such as pearls, titanium oxides, Red 6, Red 21, Blue 1, Green 5, Orange 5 dyes, chalk, talc, iron oxides and titanated micas.

Waxes may be used in the present invention in sufficient quantities to provide provided a more traditional solid form composition. Waxes are defined as lower-melting organic mixtures or compounds of high molecular weight, solid at room temperature and generally similar in composition to fats and oils except that they contain no glycerides. Some are hydrocarbons, others are esters of fatty acids and alcohols. Waxes useful in the present invention are selected from the group consisting of animal waxes, vegetable waxes, mineral waxes, various fractions of natural waxes, synthetic waxes, petroleum waxes, ethylenic polymers, hydrocarbon types such as Fischer-Tropsch waxes, silicone waxes, and mixtures thereof wherein the waxes have a melting point between 30°C and 100°C. The specific waxes useful in the present invention are selected from the group consisting of synthetic waxes, ozokerite, jojoba esters, "Unilins", available from Petrolite Corporation, fatty alcohols from C22 to C50 and mixtures thereof. Synthetic waxes include those disclosed in Warth, Chemistry and Technology of Waxes, Part 2, 1956, Reinhold Publishing. The waxes most useful herein have melting points from about 30°C to about 115°C and are selected from the C₈ to C₅₀ hydrocarbon waxes. Such waxes include long chained polymers of ethylene oxide combined with a dihydric alcohol, namely polyoxyethylene glycol. Such waxes include carbowax available from Carbide and Carbon Chemicals company. Other synthetic waxes include long-chained polymers of ethylene with OH or other stop length grouping at end of chain. Such waxes include the Fischer-Tropsch waxes as disclosed in the text disclosed above at pages 465-469 and include Rosswax, available from Ross company and PT-0602 available from Astor Wax Company. Additional synthetic waxes include the class of alkylated polyvinyl pyrrolidones or PVP, including tricontanyl PVP (available as Gannex WP-660 from ISP Company) and PVP/Eicosene Copolymer (available as from ISP Company).

Emulsifiers may be used as coupling agents which have an affinity for the hydrophilic and hydrophobic phases of lip compositions of this invention. Emulsifiers are also useful for incorporating polar fluids such as water, propylene glycol, glycerine or mixtures thereof. Such emulsifiers include those routinely used in cosmetics and are found in the CTFA. Polar fluids such as water, glycerine, propylene glycol and mixtures thereof may also be incorporated without an emulsifier when amphiphilic materials such as polyol fatty acid polyesters are used in the composition.

Skin care active ingredients in both water soluble and water insoluble forms can be added to the lip composition. Said ingredients include fat soluble vitamins, sunscreens and pharmaceutically active ingredients. These skin care active ingredients include glycerine, zinc oxide; chamomile oil; ginko biloba extract; pyroglutamic acid, salts or esters; sodium hyaluronate; 2-hydroxyoctanoic acid; sulfur; salicylic acid; carboxymethyl cysteine, and mixtures thereof.

### EXAMPLES

Examples of compositions of the present invention are as follows:

### Example 1.

| Ingredient | Weight(%) |
|---|---|
| SPE Cottonate | 89.75 |
| SPE Behenate | 5.05 |
| Sericite¹ | 5.05 |
| Propylparaben | 0.10 |
| Ethylene Brassylate | 0.05 |

| | |
|---|---|
| 1. Sericite available from U. S. Cosmetics Corporation | |

Combine all ingredients in a vessel and heat to 90°C while stirring constantly with a propeller mixer. When the SPE Behenate has completely melted and the mixture is homogeneous, remove from heat and cool to room temperature. The mixture should be stirred constantly during cooling. Transfer the resulting fluid to individual packages.

### Example 2.

| Ingredient | Weight(%) |
|---|---|
| SPE Cottonate | 88.30 |
| SPE Behenate | 4.70 |
| Mica¹ | 4.65 |
| Propylparaben | 0.15 |
| Methyparaben | 0.15 |
| Ethylene Brassylate | 0.05 |

| | |
|---|---|
| 1. Sericite available from U.S. Cosmetics Corporation | |

Combine all ingredients in a vessel and heat to 90°C while stirring constantly with a propeller mixer. When the SPE Behenate has completely melted and the mixture is homogeneous, remove from heat and cool to room temperature. The mixture should be stirred constantly during cooling. Transfer the resulting fluid to individual packages.

### Example 3.

| Ingredient | Weight(%) |
|---|---|
| Castor Oil | 89.75 |
| Glycerin/Diethylene Glycol/Adipate Crosspolymer¹ | 5.00 |
| Ozokerite | 5.00 |
| Propylparaben | 0.10 |
| Methyparaben | 0.10 |
| Ethylene Brassylate | 0.05 |

| | |
|---|---|
| 1. available as Lexorez 100 from Inolex Chemical Company. | |

Combine all ingredients in a vessel and heat to 90°C while stirring constantly with a propeller mixer. When the ozokerite has completely melted and the mixture is homogeneous, remove from heat and cool to room temperature. The mixture should be stirred constantly during cooling. Transfer the resulting fluid to individual packages.

### Example 4.

| Ingredient | Weight(%) |
|---|---|
| SPE Cottonate | 85.85 |
| SPE Behenate | 14.00 |
| Propylparaben | 0.10 |
| Ethylene Brassylate | 0.05 |

Combine all ingredients in a vessel and heat to 90°C while stirring constantly with a propeller mixer. When the SPE Behenate has completely melted and the mixture is homogeneous, remove from heat and pour into lipstick molds. Cool to approximately -5°C before de-molding and placing in an appropriate package.

### Example 5:

| Ingredient | Weight (%) |
|---|---|
| Group A: | |
| SEFA Cottonate | 84.58 |
| SEFA Behenate | 14.36 |
| Ganex Wax WP-660¹ | 0.86 |
| Propylparaben | 0.10 |
| BHT | 0.05 |

| Group B: | |
|---|---|
| Ethylene Brassylate | 0.05 |

| | |
|---|---|
| 1. Ganex Wax available from ISP Technologies, Inc. | |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into seasoned lipstick molds. Chill the filled molds at 5°C for approximately 20 minutes. Remove the molds to ambient conditions and de-mold sticks. Place sticks in lipstick cases.

### Example 6:

| Ingredient | Weight (%) |
|---|---|
| Group A: | |
| SEFA Cottonate | 70.67 |
| SEFA Behenate | 14.13 |
| Talc | 15.00 |
| Propylparaben | 0.10 |
| BHT | 0.05 |

| Group B: | |
|---|---|
| Ethylene Brassylate | 0.05 |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into seasoned lipstick molds. Chill the filled molds at 5°C for approximately 20 minutes. Remove the molds to ambient conditions and de-mold sticks. Place sticks in lipstick cases.

### Example 7:

| Ingredient | Weight (%) |
|---|---|
| Group A: | |
| SEFA Cottonate | 83.17 |
| SEFA Behenate | 16.63 |
| Propylparaben | 0.10 |
| BHT | 0.05 |

| Group B: | |
|---|---|
| Ethylene Brassylate | 0.05 |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into seasoned lipstick molds. Chill the filled molds at 5 °C for approximately 20 minutes. Remove the molds to ambient conditions and de-mold sticks. Place sticks in lipstick cases.

### Example 8:

| Ingredient | Weight (%) |
|---|---|
| Group A: | |
| SEFA Cottonate | 75.02 |
| SEFA Behenate | 13.58 |
| Talc | 7.50 |
| Ganex Wax WP-660¹ | 0.50 |
| Propylparaben | 0.15 |
| BHT | 0.05 |

| Group B: | |
|---|---|
| Glycerin | 3.00 |
| Methylparaben | 0.15 |

| Group C: | |
|---|---|
| Ethylene Brassylate | 0.05 |

| | |
|---|---|
| 1. Ganex Wax available from ISP Technologies, Inc. | |

Combine Group A ingredients together and mix well with a spatula. Heat the Group A mixture until all solids melt (approx. 90°C), stirring occasionally while heating. Combine Group B ingredients together and mix well with a spatula. Heat the Group B mixture to approximately 90°C. Combine Group A and Group B mixtures and homogenize for 5 minutes at 5000 rpm. Add Group C ingredients and mix for 5 minutes with a propeller mixer. When the mixture is homogeneous, pour the molten material into seasoned lipstick molds. Chill the filled molds at 5°C for approximately 20 minutes. Remove the molds to ambient conditions and de-mold sticks. Place sticks in lipstick cases.

### Example 9:

| Ingredient | Weight (%) |
|---|---|
| Group A: | |
| SEFA Cottonate | 59.55 |
| SEFA Behenate | 12.50 |
| Talc | 7.50 |
| Propylparaben | 0.15 |
| Vitamin E Linoleate | 0.10 |

| Group B: | |
|---|---|
| Water | 10.00 |
| Propylene Glycol | 5.00 |
| Glycerin | 5.00 |
| Methylparaben | 0.15 |

| Group C: | |
|---|---|
| Ethylene Brassylate | 0.05 |

Combine Group A ingredients together and mix well with a spatula. Heat the Group A mixture until all solids melt (approx. 90°C), stirring occasionally while heating. Combine Group B ingredients together and mix well with a spatula. Heat the Group B mixture to approximately 90 °C. Combine Group A and Group B mixtures and homogenize for 2 minutes at 5000 rpm. Add Group C ingredients and mix for 5 minutes with a propeller mixer. When the mixture is homogeneous, pour the molten material into seasoned lipstick molds. Chill the filled molds at 5°C for approximately 20 minutes. Remove the molds to ambient conditions and de-mold sticks. Place sticks in lipstick cases.

### Example 10:

| Ingredient | Weight (%) |
|---|---|
| Group A: | |
| SEFA Cottonate | 85.85 |
| SEFA Behenate | 14.00 |
| Propylparaben | 0.10 |

| Group B: | |
|---|---|
| Ethylene Brassylate | 0.05 |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into seasoned lipstick molds. Chill the filled molds at 5°C for approximately 20 minutes. Remove the molds to ambient conditions and de-mold sticks. Place sticks in lipstick cases.

### Example 11:

| Ingredient | Weight (%) |
|---|---|
| Group A: | |
| SEFA Cottonate | 85.21 |
| SEFA Behenate | 14.09 |
| Ganex Wax WP-660¹ | 0.50 |
| Propylparaben | 0.10 |
| BHT | 0.05 |

| Group B: | |
|---|---|
| Ethylene Brassylate | 0.05 |

| | |
|---|---|
| 1. Ganex Wax available from ISP Technologies, Inc. | |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into seasoned lipstick molds. Chill the filled molds at 5°C for approximately 20 minutes. Remove the molds to ambient conditions and de-mold sticks. Place sticks in lipstick cases.

### Example 12:

| Ingredient | Weight (%) |
|---|---|
| Group A: | |
| SEFA Cottonate | 89.75 |
| SEFA Behenate | 5.05 |
| Mica | 5.05 |
| Propylparaben | 0.10 |

| Group B: | |
|---|---|
| Ethylene Brassylate | 0.05 |

Combine Group A ingredients together and mix well with a spatula. Heat the Group A mixture until all solids melt (approx. 90°C), stirring occasionally while heating. Add Group B ingredients and mix for 5 minutes with a propeller mixer. Do not let the temperature rise above 90°C. When the mixture of Groups A and B ingredients is homogeneous, pour the molten material into individual containers. Allow to cool to ambient conditions.

The following are non-all encompassing examples of cosmetic products that may be used with the above complementary compositions of the present invention:

### Example 1: Lip Cosmetic Product

| Ingredients | Weight(%) |
|---|---|
| Group A: | |
| Silicone Gum¹ | 12.60 |
| Isododecane² | 12.60 |

| Group B: | |
|---|---|
| Isododecane² | 43.38 |
| Bentonite Clay⁴ | 1.00 |
| Propylene Carbonate | 0.32 |
| Red #6 Calcium Lake | 1.00 |
| Red #7 Barium Lake | 3.00 |
| Titanium Dioxide | 1.50 |
| Mica | 2.20 |
| Organosiloxane resin³ | 22.40 |

| | |
|---|---|
| 1. 2,500,000 cSt Dimethicone Gum available as SE 63 from General Electric. | |
| 2. Permethyl 99A available from Permethyl Corp. | |
| 3. MQ Resin (0.7:1 ratio M:Q) available as 1170-002 from General Electric. | |
| 4. Bentone 38 available from Rheox. | |

Combine Group A ingredients together in a beaker and mix with a propeller mixer until uniform. Combine all Group B ingredients except the propylene carbonate and hand-mix to roughly incorporate the dry powders. Homogenize the entire formulation using a Ross ME 100 LC homogenizer at about 7500 rpm until all pigments are fully dispersed. Next, while continuing the homogenization process, slowly add the propylene carbonate until mixture thickens. Combine Group A mixture with Group B mixture in a beaker and mix with a propeller mixer until uniform. Transfer the resulting fluid to individual packages.

### Example 2: Liquid Foundation Cosmetic Product

| Ingredient | Weight (%) |
|---|---|
| Group A: | |
| Organosiloxane Resin¹ | 4.48 |
| Cyclomethicone² | 11.11 |
| Silicone-polyether Emulsifier³ | 10.00 |

| Group B: | |
|---|---|
| Silicone-Treated Titanium Dioxide | 6.50 |
| Silicone-Treated Yellow Iron Oxide | 0.28 |
| Silicone-Treated Red Iron Oxide | 0.15 |
| Silicone-Treated Black Iron Oxide | 0.06 |

| Group C: | |
|---|---|
| 2,500,000 cSt Silicone Gum⁴ | 2.52 |
| Cyclomethicone² | 4.89 |

| Group D: | |
|---|---|
| Water | 49.50 |
| Glycerin | 10.00 |
| Methyl Paraben | 0.20 |
| 2-Phenoxyethanol | 0.30 |

| | |
|---|---|
| 1. MQ Resin available as 1170-002 from General Electric. | |
| 2. Cyclomethicone available as 245 fluid from Dow Corning. | |
| 3. Silicone-Polyether Emulsifier available as DC3225C from Dow Coming. | |
| 4. Dimethicone Gum (2,500,000 cSt) available as SE63 from General Electric. | |

Combine Group A and Group B ingredients together and homogenize at 9500 rpm for 15 minutes. Add Group C ingredients and homogenize at 2000 rpm for 2 minutes. Combine Group D ingredients in a separate container and mix with a propeller mixer until a clear solution forms. Add the Group D solution to the mixture of Groups A, B, and C very slowly while homogenizing at 2000 rpm. When all of the Group D solution has been incorporated, homogenize the entire mixture at 2000 rpm for an additional 10 minutes. Finally, homogenize the entire mixture at 5000 rpm for 5 minutes. Transfer the resulting fluid to individual packages.

### Example 3: Mascara Cosmetic Product

| Ingredient | Weight (%) |
|---|---|
| Group A: | |
| Organosiloxane Resin¹ | 9.60 |
| Cyclomethicone² | 8.82 |
| Silicone-polyether Emulsifier³ | 10.00 |

| Group B: | |
|---|---|
| Silicone-Treated Black Iron Oxide | 5.00 |

| Group C: | |
|---|---|
| 2,500,000 cSt Silicone Gum⁴ | 5.40 |
| Cyclomethicone² | 16.19 |

| Group D: | |
|---|---|
| Water | 43.50 |
| Sodium Chloride | 1.00 |
| Methyl Paraben | 0.20 |
| 2-Phenoxyethanol | 0.30 |

| | |
|---|---|
| 1. MQ Resin available as 1170-002 from General Electric. | |
| 2. Cyclomethicone available as 244 fluid from Dow Coming. | |
| 3. Silicone-Polyether Emulsifier available as DC3225C from Dow Corning. | |
| 4. Dimethicone Gum (2,500,000 cSt) available as SE63 from General Electric. | |

Combine Group A and Group B ingredients together and homogenize at 9500 rpm for 15 minutes. Add Group C ingredients and homogenize at 2000 rpm for 2 minutes. Combine Group D ingredients in a separate container and mix with a propeller mixer until a clear solution forms. Add the Group D solution to the mixture of Groups A, B, and C very slowly while homogenizing at 2000 rpm. When all of the Group D solution has been incorporated, homogenize the entire mixture at 2000 rpm for an additional 10 minutes. Finally, homogenize the entire mixture at 5000 rpm for 5 minutes. Transfer the resulting fluid to individual packages.

### Example 4: Shear Lip Tint Cosmetic Product

| Ingredients | Weight(%) |
|---|---|
| Group A: | |
| Silicone Gum¹ | 11.88 |
| Isododecane² | 54.45 |

| Group B: | |
|---|---|
| Organosiloxane resin³ | 20.78 |
| Red #6 Calcium Lake | 0.50 |
| Red #7 Barium Lake | 0.50 |
| Gemtone Sunstone⁵ | 0.50 |
| Timiron MP-115 Pearl⁶ | 0.50 |
| Bentone Gel⁴ | 10.89 |

| | |
|---|---|
| 1. 2,500,000 cSt Dimethicone Gum available as SE 63 from General Electric. | |
| 2. Permethyl 99A available from Permethyl Corp. | |
| 3. MQ Resin (0.7:1 ratio M:Q) available as 1170-002 from General Electric. 4. VS-5 PC available from Rheox. | |
| 5. Gemtone Sunstone available from Mearl Corporation. 6. Timiron MP-115 Pearl available from Mearl Corporation. | |

Combine Group A ingredients together in a beaker and mix with a propeller mixer until uniform. Add Group B ingredients to the Group A mixture and hand-mix to roughly incorporate the dry powders. Homogenize the entire formulation until all pigments are fully dispersed. Transfer the resulting fluid to individual packages.

### Example 5: Liquid Eye Liner Cosmetic Product

| Ingredient | Weight (%) |
|---|---|
| Group A: | |
| Organosiloxane Resin¹ | 8.90 |
| Isododecane² | 14.90 |

| Group B: | |
|---|---|
| Black Iron Oxide | 20.00 |
| Propylparaben | 0.10 |

| Group C: | |
|---|---|
| 100,000 cSt Silicone Fluid³ | 11.10 |
| Isododecane² | 33.00 |

| Group D: | |
|---|---|
| Isododecane² | 10.00 |
| Trihydroxystearin | 2.00 |

| | |
|---|---|
| 1. MQ Resin (0.7:1 ratio M:Q) available as 1170-002 from General Electric. | |
| 2. Permethyl 99A available from Permethyl Corp. | |
| 3. Dimethicone Fluid (100,000 cSt) available from General Electric. | |

Combine Group A ingredients together and mix with a propeller mixer until uniform. Add Group B ingredients and homogenize until pigments are fully dispersed. Premix Group C ingredients in a separate container using a propeller mixer until uniform, then combine with the mixture of Groups A and B ingredients. Premix Group D ingredients with heating to about 57-60°C for about 3 minutes. Remove from the heat and homogenize for approximately 5 minutes or until a gel develops. Finally, add the Group D mixture to the rest of the batch and heat the entire mixture to 57-60°C for about 7-10 minutes while mixing with a propeller mixer. Remove the batch from the heat and allow it to cool to room temperature while mixing with a propeller mixer. Transfer the resulting fluid to individual packages.

### Example 6: Eye Shadow Cosmetic Product

| Ingredient | Weight (%) |
|---|---|
| Group A: | |
| Organosiloxane Resin¹ | 22.14 |
| Isododecane² | 14.90 |

| Group B: | |
|---|---|
| Flamenco Gold Pearl | 0.60 |
| Flamenco Superpearl | 0.84 |
| Titanium Dioxide | 0.94 |
| Gemtone Copper | 0.41 |
| Gemtone Sunstone | 1.21 |
| Propylparaben | 0.10 |

| Group C: | |
|---|---|
| 1,000 cSt Silicone Fluid³ | 13.86 |
| Isododecane² | 33.00 |

| Group D: | |
|---|---|
| Isododecane² | 10.00 |
| Trihydroxystearin | 2.00 |

| | |
|---|---|
| 1. MQ Resin (0.7:1 ratio M:Q) available as 1170-002 from General Electric. | |
| 2. Permethyl 99A available from Permethyl Corp. | |
| 3. Dimethicone Fluid (1,000 cSt) available from General Electric. | |

Combine Group A ingredients together and mix with a propeller mixer until uniform. Add Group B ingredients and homogenize until pigments are fully dispersed. Premix Group C ingredients in a separate container using a propeller mixer until uniform, then combine with the mixture of Groups A and B ingredients. Premix Group D ingredients with heating to about 57-60°C for about 3 minutes. Remove from the heat and homogenize for approximately 5 minutes or until a gel develops. Finally, add the Group D mixture to the rest of the batch and heat the entire mixture to 57-60 °C for about 7-10 minutes while mixing with a propeller mixer. Remove the batch from the heat and allow it to cool to room temperature while mixing with a propeller mixer. Transfer the resulting fluid to individual packages.

### METHOD FOR IMPROVING COSMETIC PRODUCTS

The present invention covers a method of improving transfer resistant, flexible film-forming cosmetic product wherein said method comprises the steps of:
a. applying a transfer resistant, flexible film-forming cosmetic product to the skin wherein said cosmetic product has a solubility parameter less than or equal to 8.5 (calories /cm³)^{1/2};
b. allowing said cosmetic product to dry; and
c. applying over said cosmetic product a complementary composition wherein said composition has a C log P value greater than or equal to 13.

The user applies both the cosmetic product and the complementary composition of the present invention from a suitable cosmetic applicator. One such applicator used for fluid products is a liquid pen package disclosed in British Patent 21198037, issued 5/09/90, assigned to Mitsubishi Pencil Co., Ltd. of Japan.

Another such cosmetic dispenser is a unidirectional twist-up dispensing device with incremental dosing. Such a twist-up dispensing device can include a hollow housing defining a chamber having an open dispensing end and a piston located within the chamber being limited to translational movement within the chamber. The piston preferably having a threaded rod extending therefrom that engages with a threaded aperture in an actuator such that advancement of the piston toward the dispensing end occurs when the actuator is rotated. Rotation of the actuator causes the product to be dispensed from the dispensing end. An applicator is preferably attached to the dispensing end of the housing in fluid communication with the chamber wherein the product is dispensed through the applicator. The applicator can comprise a ferrule and an application portion wherein the ferrule is attached to the dispensing end of the housing and the application portion has at least one orifice located therein. Several versions of applicators can be utilized including, for example, a fiber brush or an application surface having flocking thereon. Flocking is a mat of thin, short, plastic fibers substantially perpendicular to the application surface. The bristles of a fiber brush are preferably tapered and made of a plastic material. In addition, the complimentary composition may be formed into a solid and be delivered in a more traditional applicator or implement known in the art.

## Claims

1. A method for improving the performance of long-wearing cosmetic products wherein the method comprises the steps of:
a. applying a transfer resistant, flexible film-forming cosmetic product wherein said cosmetic product has a solubility parameter less than or equal to 8.5 (calories /cm³)^{1/2};
b. allowing said cosmetic product to dry; and
c. applying over said cosmetic product a composition comprising oils having a C log P value of no less than 13.

2. A method for improving the performance of long-wearing lip cosmetic products wherein the method comprises the steps of:
a. applying on the lip a transfer resistant, flexible film-forming cosmetic product wherein said cosmetic product has a solubility parameter less than or equal to 8.5 (calories /cm³)^{1/2};
b. allowing said lip cosmetic product to dry; and
c. applying over said lip cosmetic product a composition comprising oils having a C log P value of no less than 13.

3. A method for improving the performance of long-wearing cosmetic products according to any of the preceding Claims wherein the composition applied on Step c) comprises at least 55% oil selected from the group consisting of polyol fatty acid polyesters, triglycerides, synthetic polymer oils, and mixtures thereof, preferably at least 65% polyol fatty acid polyesters, wherein the polyol fatty acid polyesters comprise fatty acid polyesters, preferably derived from any aliphatic or aromatic polyol that has at least 4 free hydroxyl groups, of which at least 80% of these free hydroxy groups are esterified with one or more fatty acids having from 8 to 22 carbon atoms.

4. A method for improving the performance of long-wearing cosmetic products according to Claim 3 wherein the polyol is a sugar polyol selected from the group of mono-, di, and polysaccharides, preferably sucrose, more preferably at least 85% sucrose polyol.

5. A method for improving the performance of long-wearing cosmetic products according to Claim 3 wherein the oils are triglycerides, preferably plant derived oils.

6. A method for improving the performance of long-wearing cosmetic products according to Claim 5 wherein the plant derived oils are selected from the group consisting of such as soy bean oil, castor bean oil, olive oil, sunflower oil, almond oil, peanut oil, canola oil, corn oil, other similarly related vegetable oils and mixtures thereof.

## Patentansprüche

1. Verfahren zur Verbesserung der Leistungsfähigkeit langgetragener kosmetischer Produkte, wobei das Verfahren die Schritte umfaßt:
a. Aufbringen eines übertragungsbeständigen, flexiblen, filmbildenden kosmetischen Produkts, wobei das kosmetische Produkt einen Löslichkeitsparameter von weniger als oder gleich 8,5 (Kalorien/cm³)^{1/2} aufweist;
b. Trocknenlassen des kosmetischen Produkts; und
c. Aufbringen über dem kosmetischen Produkt einer Zusammensetzung, umfassend Öle mit einem C log P-Wert von nicht weniger als 13.

2. Verfahren zur Verbesserung der Leistungsfähigkeit langgetragener Lippenkosmetikprodukte, wobei das Verfahren die Schritte umfaßt:
a. Aufbringen auf die Lippe eines übertragungsbeständigen, flexiblen, filmbildenden kosmetischen Produkts, wobei das kosmetische Produkt einen Löslichkeitsparameter von weniger als oder gleich 8,5 (Kalorien/cm³)^{1/2} aufweist;
b. Trocknenlassen des Lippenkosmetikprodukts; und
c. Aufbringen über dem Lippenkosmetikprodukt einer Zusammensetzung, umfassend Öle mit einem C log P-Wert von nicht weniger als 13.

3. Verfahren zur Verbesserung der Leistungsfähigkeit langgetragener kosmetischer Produkte nach mindestens einem der vorangehenden Ansprüche, wobei die beim Schritt c) aufgebrachte Zusammensetzung mindestens 55% Öl umfaßt, gewählt aus der Gruppe, bestehend aus Polyol-Fettsäurepolyestern, Triglyceriden, synthetischen Polymerölen und Mischungen hiervon, vorzugsweise mindestens 65% Polyolfettsäurepolyester, wobei die Polyolfettsäurepolyester Fettsäurepolyester umfassen, vorzugsweise abgeleitet aus irgendeinem aliphatischen oder aromatischen Polyol mit mindestens 4 freien Hydroxylgruppen, von denen mindestens 80% dieser freien Hydroxylgruppen mit einer oder mehreren Fettsäuren mit 8 bis 22 Kohlenstoffatomen verestert sind.

4. Verfahren zur Verbesserung der Leistungsfähigkeit langgetragener kosmetischer Produkte nach Anspruch 3, wobei das Polyol ein Zuckerpolyol ist, gewählt aus der Gruppe der Mono-, Di- und Polysaccharide, vorzugsweise Saccharose, weiter vorzugsweise mindestens 85% Saccharosepolyol.

5. Verfahren zur Verbesserung der Leistungsfähigkeit langgetragener kosmetischer Produkte nach Anspruch 3, wobei die Öle Triglyceride sind, vorzugsweise von Pflanzen abgeleitete Öle.

6. Verfahren zur Verbesserung der Leistungsfähigkeit langgetragener kosmetischer Produkte nach Anspruch 5, wobei die von Pflanzen abgeleiteten Öle gewählt sind aus der Gruppe, bestehend aus Sojabohnenöl, Castorbohnenöl, Olivenöl. Sonnenblumenöl, Mandelöl, Erdnußöl, Canolaöl, Maisöl, anderen ähnlichen pflanzlichen Ölen und Mischungen hiervon.

## Revendications

1. Procédé pour améliorer les performances de produits cosmétiques à longue tenue, le procédé comprenant les étapes consistant à :
a. appliquer un produit cosmétique filmogène, souple, résistant au transfert, ledit produit cosmétique ayant un paramètre de solubilité inférieur ou égal à 8,5 (calories/cm³)^{1/2} ;
b. laisser sécher ledit produit cosmétique ; et
c. appliquer, sur ledit produit cosmétique, une composition comprenant des huiles ayant une valeur de C log P non inférieure à 13.

2. Procédé pour améliorer les performances de produits cosmétiques pour les lèvres à longue tenue, le procédé comprenant les étapes consistant à :
a. appliquer, sur les lèvres, un produit cosmétique filmogène, souple, résistant au transfert, ledit produit cosmétique ayant un paramètre de solubilité inférieur ou égal à 8,5 (calories/cm³)^{1/2} ;
b. laisser sécher ledit produit cosmétique pour les lèvres ; et
c. appliquer, sur ledit produit cosmétique pour les lèvres, une composition comprenant des huiles ayant une valeur de C log P non inférieure à 13.

3. Procédé pour améliorer les performances de produits cosmétiques à longue tenue selon l'une quelconque des revendications précédentes, la composition appliquée dans l'étape c) comprenant au moins 55 % d'une huile choisie dans l'ensemble comprenant les polyesters d'acides gras et de polyol, les triglycérides, les huiles polymères de synthèse et leurs mélanges, de préférence au moins 65 % de polyesters d'acides gras et de polyol, les polyesters d'acides gras et de polyol comprenant des polyesters d'acides gras, qui de préférence dérivent d'un polyol aliphatique ou aromatique quelconque ayant au moins 4 groupcs hydroxyle libres, dont au moins 80 % de ces groupes hydroxyle libres sont estérifiés par un ou plusieurs acides gras ayant de 8 à 22 atomes de carbone.

4. Procédé pour améliorer les performances de produits cosmétiques à longue tenue selon la revendication 3, dans lequel le polyol est un polyol de sucre choisi dans l'ensemble comprenant les mono-, les di- et les polysaccharides, de préférence le saccharose, plus particulièrement un polyol à au moins 85 % de saccharose.

5. Procédé pour améliorer les performances de produits cosmétiques à longue tenue selon la revendication 3, dans lequel les huiles sont des triglycérides, de préférence des huiles d'origine végétale.

6. Procédé pour améliorer les performances de produits cosmétiques à longue tenue selon la revendication 5, dans lequel les huiles d'origine végétale sont choisies dans l'ensemble comprenant l'huile de soja, l'huile de ricin, l'huile d'olive, l'huile de tournesol, l'huile d'amande, l'huile d'arachide, l'huile de canola, l'huile de maïs, d'autres huiles végétales apparentées et leurs mélanges.
